Europäisches Patentamt

European Patent Office

Office européen des brevets

(19)

(11) Publication number: **0 256 476 B1**

# EUROPEAN PATENT SPECIFICATION

(45) Date of publication of patent specification: **28.10.92**　(51) Int. Cl.[5]: **C12Q 1/04**, C12Q 1/14

(21) Application number: **87111551.5**

(22) Date of filing: **10.08.87**

(54) **A method of detection of infection by testing of body fluid mucopolysacharide degrading enzymes.**

(30) Priority: **11.08.86 IL 79676**

(43) Date of publication of application:
**24.02.88 Bulletin 88/08**

(45) Publication of the grant of the patent:
**28.10.92 Bulletin 92/44**

(84) Designated Contracting States:
**AT BE CH DE ES FR GB GR IT LI LU NL SE**

(56) References cited:
**EP-A- 0 097 904**
**EP-A- 0 178 501**
**WO-A-80/02433**
**GB-A- 2 005 410**

**CLINICAL CHEMISTRY, vol. 27, no. 9, September 1981, pages 1490-1498, Easton, Pennsylvania, US; R.H. YOLKEN: "Enzymic analysis for rapid detection of microbial infection in human body fluids: An overview"**

**CHEMICAL ABSTRACTS, vol. 85, 2nd August 1976, page 138, abstract no. 29816m, Columbus, Ohio, US; Y. KANEKO: "Mucopolysaccharides and their degradation enzymes. Expectation for microbial enzymes", & KAGAKU TO SEIBUTSU 1976, 14**

(3), 155-7

**CHEMICAL ABSTRACTS, vol. 70, 28th April 1969, page 5, abstract no. 74242x, Columbus, Ohio, US; S. SUZUKI: "Structural studies of mucopolysaccharides by the use of degrading enzymes", & TAMPAKUSHITSU KAKUSAN KOSO 1969 14(1), 55-65**

(73) Proprietor: **Romano, Amalia**
**44, Kehilat Sofia Street**
**Tel-Aviv 69018(IL)**

(72) Inventor: **Romano, Amalia**
**44, Kehilat Sofia Street**
**Tel-Aviv 69 018(IL)**

(74) Representative: **Wuesthoff, Franz, Dr.-Ing. et al**
**Wuesthoff & Wuesthoff Patent- und Rechtsanwälte Schweigerstrasse 2**
**W-8000 München 90(DE)**

EP 0 256 476 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

### Field of Invention and Prior Art

The invention concerns a method for the in vitro diagnosis of infections and has as one of its objects a method that can be readily performed even by a person unskilled in laboratory diagnosis techniques. The diagnostic method according to the invention is applicable both in human and veterinary medicine.

Several methods are known for the in vitro diagnosis of infections, all of which require the use of well equipped laboratories and skilled personnel. Thus, for example:

1. Detection of antibodies in the blood of a patient using an in vitro antigen/antibody reaction. The main disadvantage of this method is the fact that the change in the level of antibodies occurs only after a few days from the onset of the disease, and therefore, in order to diagnose infection, repeated measurements of antibody levels have to be made over several days which causes a delay in the diagnosis and effective treatment of the disease.

2. Isolation and culturing of microorganisms from samples taken from body fluids. This method is complicated and time consuming and requires the observance of strict conditions, and even so failure of isolation occurs frequently, especially where the microorganism is a virus.

The method according to the invention is based on the observation that upon infection, mucopolysaccharide degrading enzyme (MPDE) appears in the patient's fluids such as tear fluid and cerebro-spinal fluid (CSF), where it is normally not present, and its level increases in body fluids, such as blood, in which it is normally present.

### General Description of the Invention

In accordance with the present invention, there is provided an in vitro method of diagnosing infection in a subject, comprising applying at least one sample of body fluid taken from the subject onto at least one matrix which comprises a substrate degradable by MPDE, and, after an incubation period, applying to the matrix a dye capable of binding selectively to said substrate or to a degradation product thereof and removing excess dye from the matrix, whereby a color pattern is obtained on the matrix indicative of MPDE in the tested body fluid.

The method according to the invention is suitable for diagnosis of infections in general and for early diagnosis in particular, since contrary to a change of antibody level after the onset of infection which takes a few days, the change in MPDE level occurs shortly after the onset of the disease. The method is, moreover, simple and does not require the use of elaborated laboratory equipment. Due to all this the method according to the invention is especially suitable for use in a doctor's office for quick accurate, diagnosis.

The matrix may, if desired, comprise an inert gelling agent such as agar or agarose, and the application thereto of a sample body fluid to be tested may be effected either directly or indirectly with the intermediary of an absorbent carrier soaked with said body fluid. When applying a sample directly, a drop may be placed at some point on the matrix or the sample may be spread on part of or the entire surface of the matrix. Where the body fluid sample is applied with the intermediary of an absorbent carrier, the carrier is first soaked with the sample and is then placed onto the matrix. The absorbent carrier may be of any suitable type such as a swab (which comprises a bit of an absorbent material, such as cotton wool, mounted on the tip of a small rod), a paper strip such as the SCHIRMER (trade name) tear paper test strip, a disk of an absorbent paper and the like.

The MPDE degradable substrate in the matrix may be any suitable mucopolysaccharide-glucosamine glycane such as hyaluronic acid (of various sources), chondroitin, chondroitin sulfate A, chrondroitin sulfate B (also known as dermatan sulfate), chondroitin sulfate C, chondroitin 4-sulfate, chondroitin 6-sulfate, heparan sulfate, heparin, keratan sulfate and the like.

MPDE from different sources have different specificities for different substrates and this feature may be used to advantage for diagnosing the exact type of microorganism by applying body fluid samples from the same patient to different matrices having each a different MPDE substrate.

The matrix to which the body fluid sample is applied may optionally comprise a medium on which non-viral microorganisms, (i.e., bacteria, protozoa and fungi) can grow. It is known that under appropriate conditions or under induction, such growth is accompanied by MPDE secretion and this adds to the MPDE already present in the tested body fluid and thus results in an enhanced degradation of mucopolysaccharides and, as a consequence, a more pronounced color pattern is obtained. In contrast, in case of a viral infection the amount of MPDE is only the one initially withdrawn with the body fluid sample and therefore a less prounced degradation is obtained. Thus, by using such a matrix one may differentiate between viruses and other microorganisms, which as opposed to viruses are capable of self growth.

Hereinafter, the term "growth matrix" will denote a matrix according to the invention which comprises a microorganismal growth medium and the term "basic matrix" will denote a matrix according to the invention which does not comprise such a growth medium. The invention will thus be described in the following in relation to two modes of operation: the "growth mode" in which a growth matrix is used and the "basic mode" in which a basic matrix is used.

Practically any microorganismal growth medium can be incorporated in a growth matrix, as, for example, Brain - Heart extract media, beef extract media, milk protein media, yeast extract media and the like. Any of the above media are suitable also for fungal growth, but particularly suitable growth media for that purpose being, for example, Bacto sabro liquid medium (DIFCO, 0382) or Sabro liquid medium (OXOID, SM 147).

The visual detection of infection in accordance with the present invention is based on the formation of a color pattern in the manner specified. It was surprisingly found in accordance with the present invention, that in many instances the color pattern obtained is characteristic of the specific type of microorganism causing the infection. It was further found in accordance with the invention that the formation of such differentiated and specific color patterns is manifest in particular when the body fluid is applied onto the matrix via an absorbent carrier. While the reasons for these phenomena have not yet been fully elucidated, they are used to advantage in accordance with the invention.

A typical example for the application of the invention is the diagnosis of eye infections and determination of the type of such infection, by testing for MPDE in tear fluid. Hereinafter the invention will at times be specifically described with reference to the diagnosis of eye diseases, it being understood that it is not limited thereto.

In order to diagnose an eye infection in accordance with the present invention, tear fluid is first withdrawn from the eye in a conventional manner using, for example, a micro capillary tube or an absorbent carrier such as a swab or a SCHIRMER test strip. Preferably, the tested eyes should not have been subjected to other tests, examinations or treatments at least several hours prior to the withdrawall of tear fluid for the performance of a test according to the invention, since such tests, examinations and treatments often involve the addition of solutions which effect the results obtained by the method according to the invention. The withdrawn tear fluid sample, which may first be diluted or incubated for some time in a suitable medium, is applied onto a dish containing a basic or a growth matrix. The application of the tear fluid may be performed either directly or via an absorbent carrier. If the tear fluid had been withdrawn from the eye with an absorbent carrier, the same may be used for applying it onto the matrix or the fluid may be eluted from the carrier and then be applied onto a matrix for testing as specified.

Practically, it may not always be possible to perform the test immediately after withdrawal of the body fluid by the physician or paramedic. It was found in accordance with the present invention that the activity of the MPDE in body fluid such as tear fluid is maintained over periods of time. Accordingly, in the performance of the basic mode, it is possible to store the body fluid sample, either by itself or mixed with a suitable medium, for a period of time and perform the test only thereafter. For storage the fluid is either frozen, e.g. to about -20°C, or soaked onto an absorbent carrier, which is dried and kept under sterile, non hermetic conditions and stored at a temperature of about 4°-8°C.

In many instances, a body fluid sample is stored and thereafter tested for infections therein in accordance with both modes of the invention. For such instances the body fluid sample is best stored by introducing it into a medium capable of keeping microorganisms substantially without growth and proliferation, media of this kind being known per se. Addition of the body fluid sample into the medium may be effected either directly or indirectly by placing an absorbent carrier, soaked with the body fluid sample, into, a suitable vend containing said medium. Suitable media for such a purpose are, for example, ones which have a high sodium concentration (e.g. about 8% w/v) and which are devoid of nitrogen, such as, for example, the media known in the art as Hank's medium and M-199.

The growth mode of the invention differs from the basic mode in that it requires the existence of living microorganisms in the body fluid sample to be tested. Accordingly, where the growth mode is practiced, the body fluid samples to be tested should not be stored over long periods of time.

When performing the growth mode it may sometimes be desired that the matrix will comprise also factors which favor the survival, growth or proliferation of one type or group of microorganisms over others. Such a factor may, for example, be a medium on which only one particular species grows while others do not (e.g., because it lacks a growth ingredient essential for all other species); a medium which kills one particular type of microorganism; growth regulators (inhibitors or promotors); antibiotics; a selected pH level and many more. In the performance of the growth method, the use of such factors in the matrix may be advantageous for better identification of the type of microorganism.

As distinct from conventional diagnosis methods in which microorganisms are cultured and identified as such, in the performance of the growth mode of the method according to the invention the microorganisms are not identified as such but rather detected and identified indirectly on the basis of the MPDE that they secrete. Because of the high sensitivity of the color test for the detection of a MPDE substrate or a degradation product thereof, the required incubation time needed in accordance with the invention is much shorter than in the culturing of microorganisms in accordance with the prior art (several hours as against several days).

In both the basic and growth modes, after an adequate incubation period the presence of the MPDE in the sample is determined by means of a dye that binds either to the intact substrate or to a degradation product thereof. If the body fluid such as tear fluid is applied with the intermediary of an absorbent carrier, the carrier should be removed prior to addition of the dye.

After an appropriate time during which the dye binds selectively as specified, any excess unbound dye is removed. Where the dye binds to the intact substrate, an undyed zone within and around the area to which the tear fluid sample has been applied is indicative of the presence of MPDE in the tear fluid, and accordingly of infection. Where the dye binds to a degradation product, a dyed zone or a color change within and around this area is indicative of the presence of MPDE in the tear fluid and accordingly of infection.

MPDE from various substrates differ not only in their specificity for various substrates (as stated above) but also in the nature of the degradation products obtained from one and the same substrate. This feature may be used in accordance with the present invention to identify a particular MPDE, and thereby determine the pathogen from which the so identified MPDE is derived. Such an identification may be performed by employing a dye, or a mixture of dyes, which yield different colors with different degradation products; or a dye which binds selectively to a particular degradation product. Thus, for example, MPDE derived from the human body, degrades mucopolysaccharides into tetramers or octamers of the basic sugar units whereas a bacterial MPDE degrades mucopolysaccharides into dimers (Ludowieg, J. et al., 1961, J. Biol. Chem. 236: 333-339). As a viral infection merely stimulates the secretion of human MPDE, detection of dimers, is conclusive of a bacterial infection and consequently by using a dye that binds selectively to dimers it is possible to differentiate, between viral and bacterial infections. For example, it was found in accordance with the present invention, that MPDE substrate is degraded into octamers by the MPDE present in the tear fluid upon occurance of a viral infection, in particular in case of an herpetic eye infection, and a dye which binds to octamers may thus serve for the diagnosis of such infections.

As already explained above, when body fluid samples are tested in accordance with the invention using a dye that binds to the intact substrate, the existence of an infection, whether viral or bacterial, is diagnosed by the appearance of a discolored zone on those parts of the matrix where MPDE was degraded; and where the dye binds to a degradation product of the substrate the resulting color pattern will be characterized by the appearance of a colored zone at the parts of the matrix where MPDE was degraded; and that specific color patterns obtained are indicative of the type of infection. It was moreover found in accordance with the present invention that there exists a correlation between the degree of infection and the MPDE level in the various body fluids. Thus, the degree of infection may be determined by the extent of the discoloration or coloration, depending on whether the employed dye binds to intact substrate or to a degradation product thereof.

Therefore, in accordance with a particular aspect of the invention there are provided charts of color patterns for different infectious diseases and severity thereof as well as for different dyes and substrates. With the aid of such charts, even a person unskilled in laboratory techniques, can determine the type of infection by comparing the color pattern obtained with those in the chart or charts. Such a determination is unique in that it does not require the isolation of the microorganism that causes the infection. Moreover, the size and intensity of the discolored zone is indicative of the degree of infection.

The color patterns which are obtained are so characteristic of the type of infections that these may also be diagnosed with the aid of a computerized images analysis system (Cue 2 Image Analysis System, Olympus Corporation New York, USA) and characteristic blue-gray, red-gray and green-gray level histograms are obtained depending on the type of infection.

If desired, the basic and growth modes may both be simultaneously or sequentially used for determining the existence, degree and type of infection.

For determining the existence and degree of infection, the basic mode is especially suitable. The appearance on the basic matrix of a colored or discolored zone, as the case may be and depending on whether the dye binds the intact substrate onto a degradation product thereof, is evidence of the existance of MPDE in the tested body fluid sample. In case of body fluids in which MPDE is normally not present, such as tear fluid and CSF and where the dye binds to the intact substrate, a discolored zone is indicative of an infection, while in other body fluids that normally contain MPDE, such as the blood serum, the extent of the discolored zone will be measured and compared to the discoloration obtained from normal sera. Thus, for example, it was found that when a disk of absorbent paper soaked with 30 $\mu$l of a normal blood serum is placed onto the basic matrix a circular discolored area is obtained having a diameter of about 12 mm, whereas this diameter is increased upon the occurance of infection.

The growth mode is especially suitable for determining whether the infection is caused by microorganisms other than viruses, since the growth of microorganisms is accompanied by MPDE secretion resulting in enhanced degradation of the substrate as compared to the degradation on a basic matrix. In case of a viral infection it was found that incubation of a body fluid sample on the growth matrix results in no or only slight degradation of the matrix which is probably due to some factors present in the growth media which inhibit the MPDE present in the body fluid sample. In contrast, the growth media was not found to inhibit MPDE secreted by microorganisms.

As already mentioned, the MPDE present in body fluids during a viral infection originates from body cells only, while in case of a bacterial or a protozoan infection MPDE originating from the microorganism itself is present in addition to that originating from body cells. MPDEs from different sources have preference towards different substrates, and such preferences may serve as further means for differentiating between viral and non-viral infectious diseases and between different types of non-viral diseases.

MPDEs from different sources have different pH sensitivity spectra. Thus, for example, MPDE from bacterial origin is most active in alkaline pH whereas cellular MPDE is most active in acidic pH. Such a different pH-dependent activity of the different MPDEs may serve as an additional means for differentiating between viral and non-viral infections.

MPDEs from different sources also differ in their temperature sensitivity. MPDE from a cellular origin, for example, is still active at temperatures above 50°C whereas a bacterial MPDE loses its activity at such temperatures. This temperature sensitivity difference may serve as yet another differentiating factor.

It was found in accordance with the invention that when a SCHIRMER paper test strip soaked with body fluids is cut longitudinally into two halfs, each one gives exactly the same color pattern when incubated on the same matrix at the same temperature. Therefore, if such two halves are incubated on different matrices, any difference in the color pattern will be due to difference in the reaction of the MPDE with the two different substrates. Thus, in the performance of the method in accordance with the present invention it is possible to withdraw a single body fluid sample onto a carrier, devide the carrier between different dishes holding different matrices and any difference in the color patterns will enable to determine the type of infection.

A typical procedure for detecting infections may involve testing a body fluid sample simultanuously in accordance with both the basic and the growth mode and if desired with the aid of charts of color patterns as specified. In such a procedure, the existence and type of infection, i.e. whether viral, bacterial or protozoal, may be determined with a high degree of accuracy within only several hours. Consequently, a patient may receive the correct therapeutical treatment, e.g. different anti-viral drugs and interferon in case of a viral infection or an antibiotic in case of a bacterial infection, very shortly after his first check. This distinguishes favorably from tedious laboratory tests where a long waiting time lasting for days and occasionally even weeks is required until the patient may receive the exact treatment he requires.

Typically, detection of an ocular infection and determination of the type of such an infection may, for example, be as follows:

(a) incubation of the tear fluid sample onto the basic matrix in order to determine the presence of an infection which in case of a dye that binds to intact substrate will be apparent by the appearance of a discolored zone; in case the test shows the presence of an infection, then

(b) placing a tear fluid sample onto the growth matrix, in order to determine whether the infection is viral, bacterial or protozoan;

(c) if desired, determining the exact type of microorganism that causes the infection, by applying tear fluid samples onto various basic and growth matrices with and without an intermediary carrier and recording the color patterns then obtained.

For carrying out the method in accordance with the invention, an assay kit is provided which comprises at least one dish containing a matrix as hereinbefore described, and a dye which binds either to the intact substrate or to a degradation product thereof. In addition, the kit may comprise a chart of color patterns to which a color pattern obtained may be compared. Where the kit comprises a plurality of matrix containing dishes, some may contain a basic matrix and some a growth matrix. Furthermore, some of the growth matrix containing dishes may also comprise growth regulators, i.e. growth promoters or growth inhibitors. Such growth regulators may also be provided separately and be added onto the matrix together with the body fluid sample.

A kit according to the invention may optionally also comprise means for withdrawing body fluids, such as glass capillaries, needles and the like and also absorbent carriers such as a sterile swab, paper strips and paper disks.

## Description of Specific Embodiments

The invention will now be specifically described in some non-limiting Examples which concern determination of infections in tear fluids it being understood that tear fluid was selected for illustration only and that the invention concerns detection of infections in body fluids in general.

Furthermore, detection of MPDE in these Examples is described with repsect to a dye that binds to the intact substrate it being understood that the invention also encompasses, mutatis mutandis, use of a dye that binds to a degradation product.

Example I: Preparation of basic matrices

**A.** Chondrotin sulfate A matrix

0.75 gm of agarose (manufactured by BDH Chemicals Ltd., England, product No. 33006) and 0.05 gm of chondroitin sulfate, type A (sodium salt, from bovine manufactured by SIGMA C-4134) are mixed with 100 ml of double distilled water (DDW). If necessary, the pH is adjusted, e.g. to 7.0, by the addition of a few drops of 1N HCl or 1N NaOH as the case may be.

For sterilization, the flask containing the mixture is otoclaved at 120°C for 20 minutes. During autoclaving, the agarose is dissolved in the DDW, by which a homogenous agarose solution is obtained.

The agarose undergoes hydrolysis when subjected to high temperatures at a low pH. Therefore, if it is desired to prepare a chondroitin sulfate matrix comprising agarose at a low pH, e.g. 4.0, it is best that two separate solutions are prepared one consisting of agarose dissolved in DDW and the other consisting of chondroitin sulfate in DDW at a desired low pH; the two solutions are thus autoclaved separately and mixed only after they had been cooled somewhat, by which a similar homogenous agarose solution, as above, is obtained.

Instead of 0.05 gm of type A, it is also possible to use 0.025 gm of chordroitin sulfate type B (sodium salt, mixed isomers, SIGMA, catalog No. C-3219).

After the agarose solution is cooled to about 50°C, 4 ml portions thereof are transferred to small culture dishes, e.g. having 5 cm diameter (such as those manufactured by UNOPLAST, Hundested, Denmark). The agarose is left to jellify at room temperature and then kept at 4°-8°C for at least 24 hours before use.

**B.** Chondroitin sulfate, A, B and C matrix

By the same procedure as in A above, Petri dishes containing chondroitin sulfate mixed isomers (chondroitin A, B and C manufactured by SIGMA C-8529, C-3129 or C-3255) matrices are prepared, but instead of chondroitin type A, the starting mixture comprises a mixture of chondroitin sulfate type A, B and C.

**C.** Hyalouronic acid matrix

0.05 gm of Hyaluronic acid (manufactured by SIGMA, catalog No. H-1751, H-1876 or H-1504, the former one is preferred) and 0.75 gm agarose are mixed with 100 m of DDW. The mixture is heated somewhat for dissolving the agarose. Thereafter the solution is otocloved as in A above, and after cooling 4 ml portions are transferred to small petri dishes.

**D.** Matrices containing other MPDE substrates

Similarly as in A to C above, matrices are prepared comprising chondroitin sulfate B (SIGMA, C-4259) or C (SIGMA C-4384), chondroitin-4-sulfate, chondrotin-6-sulfate, heparan sulfate, heparin, or keratan sulfate.

Example II: Preparation of a toluidine blue solution

A toluidine blue solution may be prepared as follows:

0.4 gm of toluidine blue are dissolved in 80 ml of acetone and into the resulting solution 920 ml of DDW are admixed. The resulting aqueous toluidine blue solution is filtered and thereafter the dye solution is ready for use.

Example III: Testing for infection in accordance with the basic mode

A tear fluid sample is withdrawn from the eye by a sterile absorbent carrier such as a swab or a SCHIRMER paper test strip, and the carrier is transferred to a sterile test tube until use. The absorbent carrier is dried for several minutes and then placed onto a matrix placed in a sterile tissue culture dish, and incubated for 3-24 hours at 37°C. The matrix may be any one of those described in Example I A to D, above.

After incubation, the carrier is removed and 4 ml of a toluidine blue solution prepared in accordance with Example II is added, followed by incubation for about 10 minutes. Thereafter, the toluidine blue solution is poured from the dish and the matrix and dish are then rinsed twice for 10 minutes with a 5% acetic acid solution. Thereafter the dishes are washed with running water and dried at room temperature.

The color which is obtained for undegraded chondroitin sulfate is purple as compared to dark blue for undegraded hyaluronic acid.

For detecting infections in general it is especially useful to employ a matrix comprising a substrate having a broad spectrum (i.e., being degradable by a wide variety of MPDEs) like the one comprising mixed isomers of chondroitin sulfate of Example I B. Substrates having a narrower spectrum, e.g. the one of Example I A, may be employed for more specific determination of the pathogen.

Example IV: Preparation of growth matrices

**A.** Brain-Heart, chondroitin sulphate - matrix

3.7 gm of Brain-Heart infusion broth (manufactured by Institution Pasteur, catalog No. 64017) and 0.025 gm of chondroitin sulfate, type BI (manufactured by SIGMA, catalog No. 3129) are dissolved in 100 ml DDW and the pH is adjusted to 8 by the addition of an adequate amount of 0.1N NaOH. Thereafter 0.75 gm of Agarose (manufactured by BDH, product No. 33006) is admixed and dissolved by heating. The resulting solution is sterilized by autoclaving at 120°C for 20 minutes.

After cooling to about 50°C, 4 ml portions are transferred to small petri dishes (as above), the agarose is left to gelify at room temperature and the dishes are then kept at 4-8°C for at least 24 hours before use.

**B.** Matrices containing other substrates and media

In a similar manner, matrices are prepared which contain growth media other than Brain-Heart extract, as follows:
1. Tryptose glucose extract Agar (manufactured by DIFCO, catalog No. 0064-01), pH adjusted to 7.4.
2. Standard plate count medium, pH adjusted to 7.0.
3. Tryptose glucose yeast extract, pH adjusted to 7.0.

**C.** Matrices with other MPDE substrates

In addition to chondroitin sulfate, the above growth matrices (A and B) are prepared with different MPDE substrates, e.g. those of Example I.

In all the above procedures A to C, agarose may be replaced by agar. Furthermore, the matrices may be prepared at different pH levels ranging from about 4.0 to about 8.5.

For better classification of the type of bacteria, the growth matrix may comprise growth inhibitors for one particular type of bacteria, e.g. bactobile salts and bacto neutral red which inhibit the growth of gram (+) and not gram (-) bacteria, such as McConkey Agar (manufactured by DIFCO, catalog No. 0075-01). In this way, it is possible to differentiate between these two groups of bacteria. Similarly, the media may contain α-phenylethylalcohol which inhibits the growth of gram (-) and not gram (+) bacteria.

Likewise, the matrix may also contain growth inhibitors and growth promoters.

**Description of the Drawings**

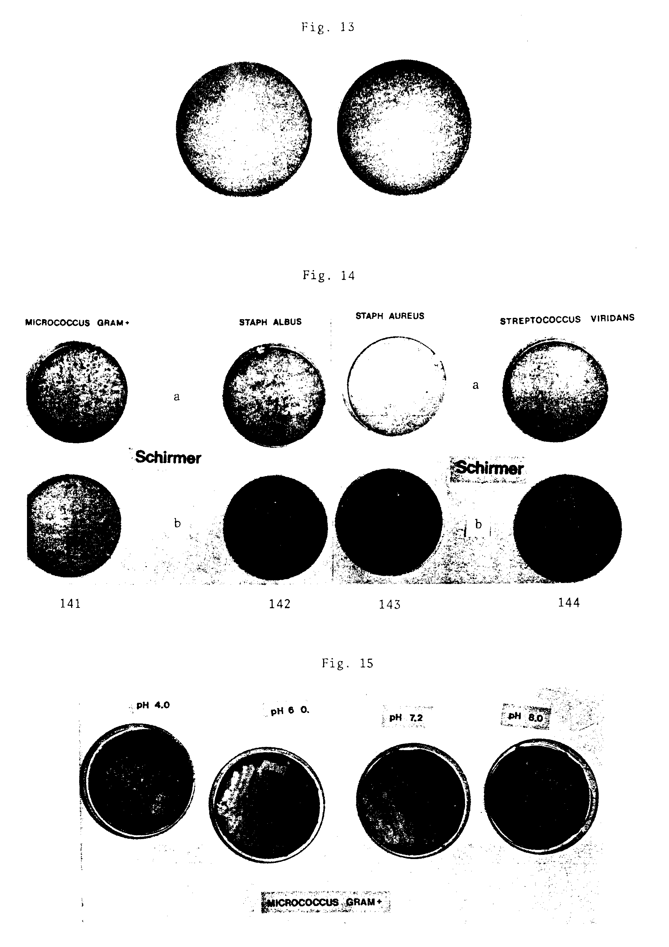

For better understanding, the invention will now be described by way of example only, with reference to the annexed drawings which show a black and white representation of various color patterns, resulting from incubation of body fluid samples from different subjects in dishes holding either a basic or a growth matrix, the substrate being chondroitin sulphate. The performance of color reactions on the dishes and development thereof was performed as described hereinbefore. It should be emphasized that in some cases and especially in the case of bacterial infections as in Fig. 14, the exact patterns is better appreciated when seeing the plates in full color.

Fig. 1 shows a dish holding a basic matrix and depicting a negative result obtained upon incubation with a 6mm antibiotic test disk of absorbent paper (manufactured by Schlecher and Schull) to which 10 ul of tear fluid from a healthy subject was applied;

Fig. 2 shows a dish holding a basic matrix and depicting a negative result obtained upon incubation with a SCHIRMER (trade name) paper strip on which tear fluid was absorbed by placing the paper strip for 5 min. in the eye of a healthy subject;

Fig. 3 shows a dish holding a basic matrix and depicting a positive result obtained upon incubation with a 6mm absorbent paper disk, of the type specified with respect to Fig. 1, on which 10 $\mu$l of tear fluid taken from a subject suffering from an ocular Adenovirus infection was applied;

Figs. 4 and 17 show a dish holding a basic matrix and depicting a positive result obtained upon incubation with a SCHIRMER (trade name) paper strip on which tear fluid was absorbed by placing the paper strip for 5 mins. in the eye of a subject suffering from an ocular Adenovirus infection;

Fig. 5 shows a dish holding a basic matrix and depicting a positive result obtained upon incubation with a 6mm absorbent paper disk on which 10 $\mu$l of tear fluid from a subject suffering from an acute ocular herpetic infection, was applied;

Figs. 6 and 18 show a dish holding a basic matrix and depicting a positive result obtained upon incubation with a SCHIRMER (trade name) paper strip, on which tear fluid was absorbed by placing the paper strip for 5 mins. in the eye of a subject suffering from an acute ocular herpetic infection;

Fig. 7 shows a follow-up of an Adenovirus bilateral infection showing improvement upon treatment with interferon; the dishes each holding a basic matrix were incubated with paper strips on which tear fluid was absorbed by placing the paper strip for 5 mins. in the eye of the same subject consecutively (from left to right) after the initiation of the treatment;

Fig. 8 shows a follow-up of an acute ocular herpatic bilateral infection on a dish holding a basic matrix, tear fluid samples were taken consecutively, after the onset of the disease, from the eyes of the same subject by placing a SCHIRMER (trade name) paper strip for 5 mins, in the eye of the subject;

Fig 9 shows a dish holding a basic matrix demonstrating a positive result obtained upon incubation with a 6mm absorbent paper disk of the above specified type, on which 10 $\mu$l of blood serum, taken from a patient suffering from herpetic encephalitis, was applied in the course of two stages of the disease;

Fig. 10 shows several dishes each holding a basic matrix and depicting a positive reactions with serum samples from a patient suffering from a general herpatic infection, the upper row showing tests performed upon onset of the infection (at several dilutions) and the lower row showing tests performed 8 days later and depicting a much reduced reaction;

Fig. 11 shows several dishes, each holding the basic matrix and depicting a positive reaction with a serum sample (two dishes on the left) and a tear fluid sample (right eye-center dishes, left eye-right dishes) from two patients (each row from a different patient) suffering from a general infection of Chlamydia (an infectious protozoa causing tracohma); serum samples were placed on the matrices by using an absorbent paper disk and the tear fluid samples by using a SCHIRMER paper strip;

Fig. 12 shows two dishes, each holding a basic matrix, depicting a positive reaction in the right eye (right dish) and a negative reaction in the left eye (left dish) in tear fluid samples withdrawn from the eyes of a patient suffering from an acute herpatic infection in his right eye. Withdrawal of tear fluid was by means of a swab which was then placed on the dish;

Fig. 13 shows a dish holding a growth matrix depicting a negative result is obtained upon direct incubation with a control fluid (i.e. containing no infectious agent);

Fig. 14 shows several dishes containing growth matrices, demonstrating different color patterns obtained with samples obtained from cultures of different bacteria species either by direct plating of a sample onto the matrix (upper row) or with the intermediary of a SCHRIMER (trade name) paper strip;

Fig. 15 shows dishes holding the growth matrix at different pH levels (as indicated above each dish) which were incubated with a fluid sample taken from a culture of micrococcus gram ( + );

Fig. 16 shows dishes, five of which hold the growth matrices at various pH levels (a - 4.0, b - 6.0, c - 7.2, d - 8.0, e - 8.5) and one holding a basic matrix (f), all incubated with a swab soaked with tear fluid withdrawn from a patient suffering from an acute micrococcus grown ( + ) infection;

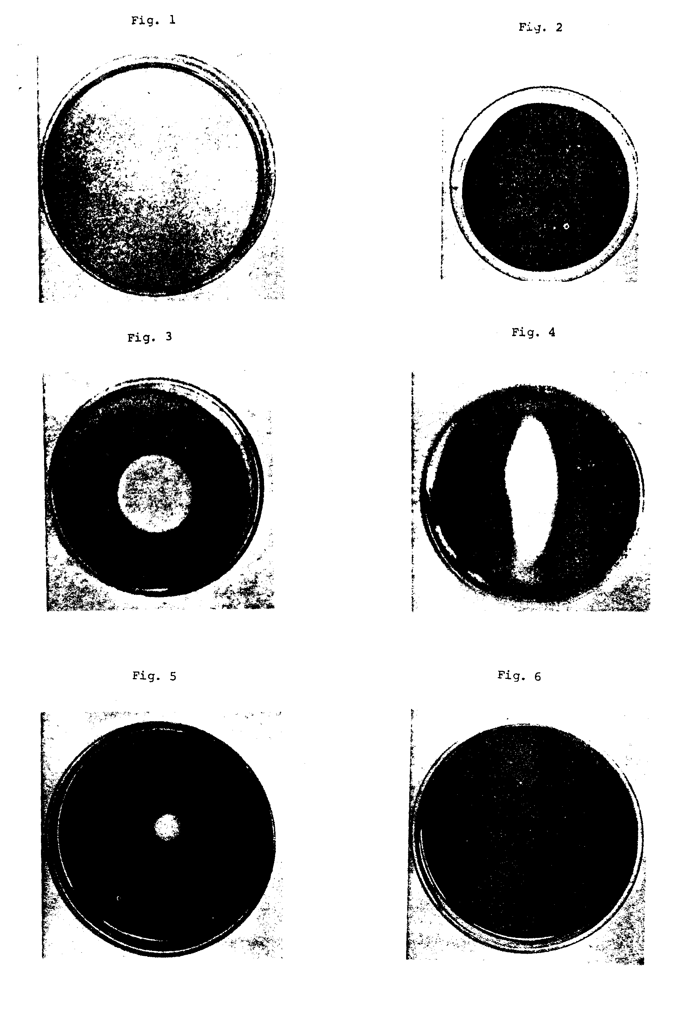

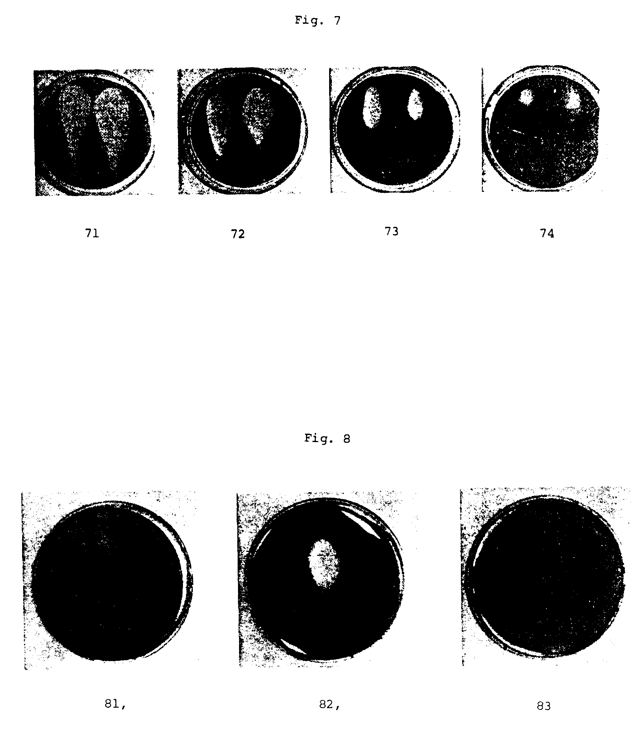

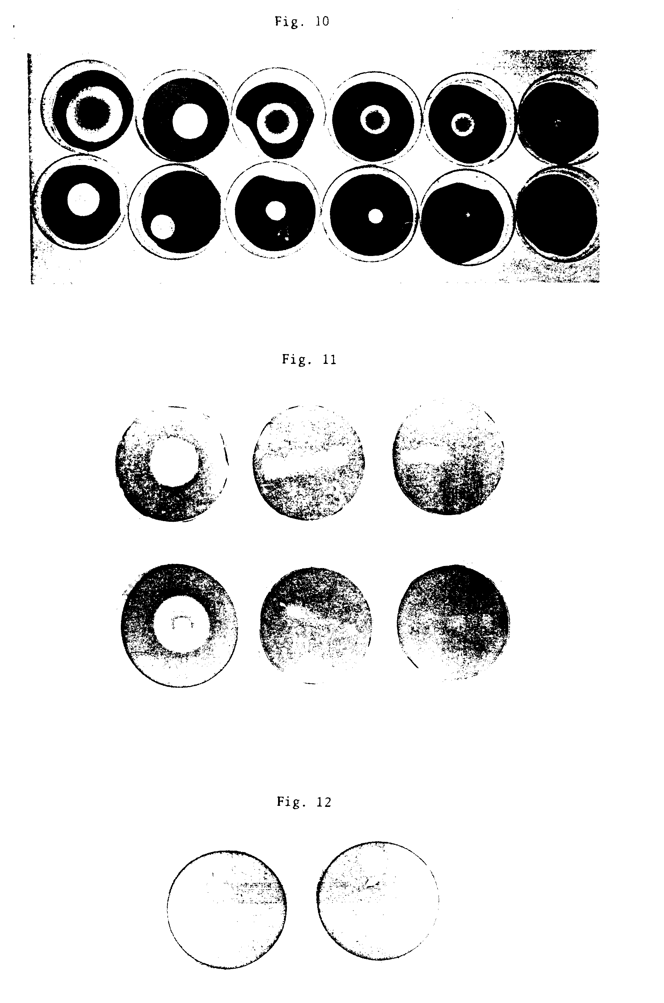

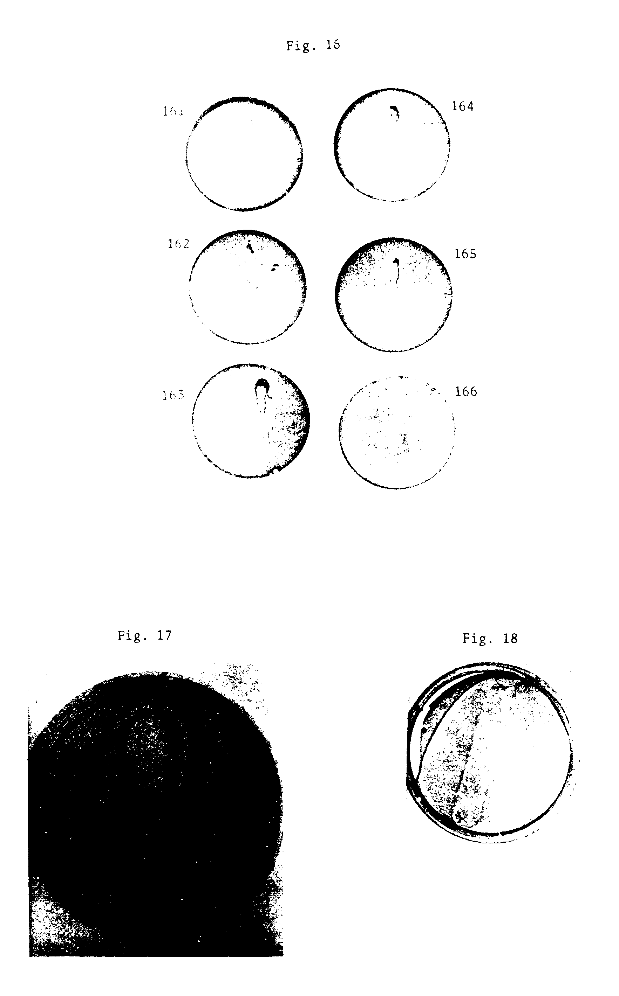

Tear fluid collected from a normal, uninfected eye and tested in accordance with the invention produced a negative result as shown in Figs. 1 and 2, for a basic matrix and in Fig. 13 for a growth matrix. Toluidine blue which binds to a glucosaminglycan-substrate, colors the whole plate. In the case of infection an MPDE is present in the tear fluid and therefore a part of the glucosamin-glycan substrate is degraded in the zone located underneath the said paper strip. Consequently, color patterns are observed on the plates shown in Figs. 3 - 8, 10 - 12, and 14 - 16 whether tear fluid is applied directly onto the matrix (Figs. 14 and 15), or indirectly with a swab (Figs. 12 and 16), an absorbent paper disk (Figs. 3, 5, 9, 10 and 11) or a SCHIRMER paper strip (Figs. 4, 6, 7, 8, 11 and 14). It should be mentioned that when a sample is placed onto the matrix by using an intermediary absorbent carrier, such as a swab, a slight decoloration is sometimes observed upon incubation with a control or uninfected sample due to physical pressure exerted by the carrier onto the matrix.

The color pattern obtained in the performance of the method according to the invention may be characteristic of the type of the infection. Thus, for example, the type of a viral infection may be determined on the basic matrix as follows:

1. Adeno Virus infection is characterized by a relatively large round discolored zone which may exceed the area covered by the absorbent carrier during incubation having a dark, well defined edges, when the infected tear fluid is applied onto a disk of an absorbent paper (Fig.

3), and an egg-like shape of the discolored zone also having well defined dark edges (Fig. 4), or a tailed oval shape (resembling the shape of a tennis racket or a chinese spoon) in the first stages of the disease (Fig. 7) when infected tear fluid is applied onto a SCHIRMER (trade name) paper strip.

2. Herpes virus infection is characterized by a small round discolored zone, which does not have a well defined edge when the infected tear fluid is applied onto a disk of an absorbent paper (Fig. 5), and a mosaic like pattern of discoloration when the infected tear fluid is applied onto a SCHIRMER (trade name) paper strip (Fig. 6). In both these cases, the discolored zone is confined to the area which during incubation was covered by the absorbent carrier. In cases of a strong herpetic infection, a full discolored zone is observed in part of the area that had been covered during incubation by the carrier (Figs. 8 and 12).

The type of a bacterial infection may, for example, be determined on a growth matrix, as follows:

3. Micrococcus gram ( + ) infection is characterized by well defined dark blue dots with a slight halo around them on a light-blue background, when the tested sample is applied uniformly over the entire surface of the matrix (item 141a in Fig. 14). When a sample is applied onto the matrix by means of a SCHIRMER test paper strip, a zone of a slight discoloration appears under the area which had been covered during incubation by the test strip, which has a periphery of well defined dark dots, concentrated particularly at the side of the paper strip which had been in contact with the body fluid sample (item 141b in Fig. 14). A similar pattern is also obtained upon incubation with a swab (items 162-165 in Fig. 16).

4. Staphilococcus albus infections are characterized by diffuse strands of coloration on a bright blue-green background, when the tested sample is applied uniformly over the entire surface of the matrix (item 142a in Fig. 14). When a sample is applied onto the matrix via a SCHIRMER test paper strip, a slightly darkened edge appears around the place covered during incubation by the paper test strip which is brighter than its surrounding (item 142b in Fig. 14).

5. Staphilococcus aureus infections are characterized by dark dots, not as well defined as those of micrococcus gram ( + ), on a non-uniform background having a slightly discolored central region when the tested sample is applied uniformly over the entire surface of the matrix (item 143a in Fig. 14). In a plate where the sample had been applied via a SCHIRMER test paper strip a discolored zone which corresponds with the area which had been covered during incubation by the test paper strip is obtained having small darker zones at its one end (item 143b in Fig. 14).

6. Strepyococcus viridans is characterized by a general yellowish discoloration of the matrix with a few darker zones having a definite organization (item 144a in Fig. 14). When the fluid sample is applied onto the matrix by means of a SCHIRMER test paper strip a discolored zone is obtained which is wider than the area which had been covered by the paper strip during incubation.

A follow up of the progress of an herpetic infection is shown in Fig. 8, where 81 shows a test at the onset of the acute infection when the infection was still relatively weak, 82 shows a test performed at an intermediary stage, when the infection was at its maximum, and plate 83 shows a test performed at the end of the acute infection when it almost disappeared.

The method according to the invention enables also to determine the severity of the infection, which is indicated by the extent and depth of the discolored zone. Depending on the severity of the infection (i.e. the concentration of MPDE in the body fluid), the interaction between MPDE and the substrate in the matrix may exceed the zone covered by the carrier during incubation. This phenomenon may be used for following the progress of therapy as shown in Fig. 7 where 71 shows a test before treatment and 74 a test at an advanced stage of the treatment of the same patient, while 72 and 73 show intermediary stages.

Determination of the severity of an infection is facilitated when different body fluid sample dilutions are used as shown in Fig. 10, wherein the dishes in the left column were incubated with sera 30 λ of undiluted samples and each successive dish at the right was incubated with a blood serum sample diluted two fold over the sample on its left (i.e. 1; 1/2; 1/4; 1/8; 1/16; 1/32.) The dark areas in the upper row of dishes is due to haemolysis of red blond cells.

The method according to the present invention is useful also in detecting infections caused by a protozoa as shown in Fig. 11 wherein the dishes were incubated with sera samples (two dishes at left) and tear fluid samples (center and right dishes) withdrawn from patients suffering from a general Chlamydia infection.

The color pattern which is obtained is affected by the pH of the medium as shown in Figs. 15 and 16. In Fig. 15, in which a control fluid sample containing micrococcus gram ( + ) bacteria was plated on the entire surface of the dish containing a growth matrix at different pH levels (indicated above the dishes) no reaction is seen at a pH of 4.0 and a positive reaction at pH of 6.0, 7.1 and 8.0. In Fig. 16, the maximal reaction is obtained at a pH of 7.2 (item 163) whereas no or very little reaction is obtained at a pH of 4.0 (item 161) and a basic growth matrix (item 166). The slight discoloration obtained in items 161 and 166 is mainly due to matrix depressions caused by the swab.

The following clinical tests in humans and in animals demonstrate the usefulness of the invention:

Test No. 1

The eyes of 2,500 subjects, i.e. 5,000 eyes, were tested using the basic mode as described hereinbefore. Almost all subjects showed a negative reaction except for an insignificant number of 30 normal subjects with particularly dry eyes who gave a false-positive reaction.

All subjects with an acute infection showed a definite positive reaction expressed by a discolored zone, as shown in the following tests 2 to 7:

Test No. 2

1,000 Subjects with bilateral Adenoviral infection, i.e. 2,000 eyes were tested in accordance with the basic mode of the invention. In the tests all infected eyes produced definite discolored zones typical of Adenovirus infections, as described above, which became manifest immediately after the onset of the disease and throughout its duration. There was a close correlation between the intensity and size of the reaction and the severity of infection.

Test No. 3

The same test was performed on 500 cases with a monocular Adenovirus infection showing an overall positive reaction typical of Adenovirus infections, as described above, in the samples taken from the infected eyes while the healthy eyes showed a negative reaction.

Test No. 4

1,000 Subjects with monocular Herpes Simplex virus infection were tested in accordance with the basic mode and showed a positive reaction of the infected eyes during the acute stage of the disease with discolored zones, typical of Herpes Virus infections, as described above.

Test No. 5

50 Cases with a bilateral Herpes Simplex infection were tested in accordance with the basic mode of the invention and both eyes showed a definite positive reaction, typical of a Herpes Virus infection, as described above, during the acute stage of the disease.

Test No. 6

In 5 patients with a recurrent Herpes Simplex Type 1 ocular infection, when testing in accordance with the basic mode of the invention, a delicate mosaic-like discoloration pattern was found during the acute stage of the herpetic disease, typical of a Herpes Virus infection, as described above. When the same patients developed an Adenovirus infection, the pattern of the discolored zone turned tailed-oval which is typical of the Adenovirus infection.

Test No. 7

In 10 cases with a unilateral ophthalmic Herpes Zoster infection, tested in accordance with the basic mode of the invention, a positive reaction with a bold mosaic-like pattern of the bright area was obtained in the infected eye, whereas the uninfected eye showed a negative result.

The following tests, 8 and 9, show the application of the invention to body fluids other than tears:

Test No. 8

Cerebro-spinal fluid samples taken from 20 patients not suffering from a viral infection in the central nervous system showed a negative result when tested by the method in accordance with the basic mode of the invention, whereas cerebro-spinal fluid samples taken from 10 patients with Herpetic Encephalitis, developed a positive reaction even in the initial stages of the infection.

Test No. 9

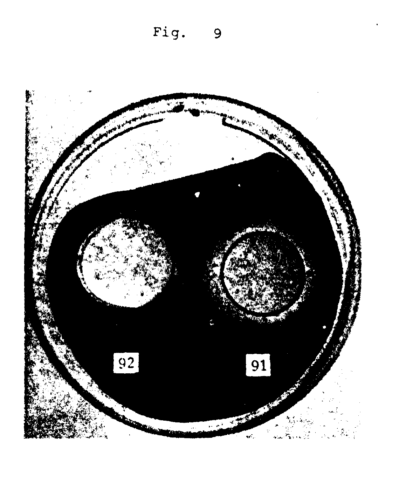

Tests according to the basic mode of the invention performed with blood sera from 50 patients with viral diseases showed a stronger reaction with a larger discolored zone having a diameter of about 16 mm, at the beginning of the viral infection (91 in Fig. 9), and a much smaller bright area, having a diameter of about 12 mm (92 in Fig. 9) at the end of the infection. Discolored zones with a diameter of about 12 mm correspond to the normal MPDE level in the blood. The study included patients with Herpes infection, Rubella, and Mumps.

Test No. 10

In grown patients suffering from a Chlamydia infection, acute large discoloration having a diameter of 19-23 mm (as compared to 11 mm in control sera) was obtained when dished containing the basic matrix were incubated with blood sera, and a positive reaction was also obtained when tear fluid samples were incubated on the matrix.

The existence of a Chlamydia infection was verified by the Immunoperoxidase method for the detection of specific IgG and IgH antibodies to Chlamydia, using a test kit manufactured by Savyon Diagnostics Ltd. (catalog No. 011-01-144).

Test No. 11

In tear fluid withdrawn from a patient known to have AIDS a positive reaction was obtained in correlation with the occurance of an acute infection and fever.

Test No. 12

In another AIDS patient a positive reaction was obtained in tear fluid which was reduced upon treatment.

Test No. 13

Tear fluid from eight rabbits were tested in accordance with the basic mode of the invention. The rabbits were divided into four groups, each group consisting of two animals, as follows:

one group with healthy, uninfected eyes served as control;

a second group had an acute herpes simplex infection;

a third group had a latent herpes simples infection, which was activated by passing an electric current between two electrodes, one being an electric needle placed in the eye and the other being an electrode attached to the ear;

a fourth group was infected with staphilococcus.

In each experimental group one eye had been infected and the other served as control.

In all cases, a characteristic positive reaction was obtained already on the first day of infection. The intensity of infection was determined by methods known in the art and was found to be in correlation with the intensity of the discoloration of the matrix (the dye used bound to intact MPDE) for all types of infection.

Test No. 14

A similar test as Test No. 13 was performed with six guinea pigs, two of which served as control, two of which had an acute herpes zoster infection and two of which had a latent herpes zoster infection which had been activated as above (Test No. 13).

Similarly as in Test No. 13 a correlation between the degree of infection and the depth of discoloration was observed.

However, in both Test No. 13 and 14, false positive results were obtained from dry eyes,i.e. from eyes which on a SCHIRMER standard test strip produced after 5 min. a 5 mm long wet zone, as compared to 25-30 mm with healthy human adults.

Test No. 15

11 rabbits infected with pseudomonas aureginosu were divided into two groups, of which one received no treatment while the other was treated against infection with a protease inhibiting substance.

Here as well a correlation between the degree of infection and depth of discoloration was obtained, and when infection was reduced by the treatment, the depth of discoloration was reduced accordingly.

In the following examples some of the assay kits are described which serve for the detection of MPDE in various body fluids, in order to determine type and degree of an infection, in accordance with the invention. In these examples each kit comprises a plurality of specimens of each item such as capillary tubes, paper strips and dishes, it being understood that a kit may also comprise only a single specimen of each item.

Example 1

An assay kit is provided comprising:

a number of capillary tubes for withdrawal of fluid to be tested and paper test strips for absorption of the fluid to be tested either directly or from the glass capillary;

micro-tissue-culture dishes holding agar or agarose matrix mixed with chondroitin sulfate (A, B or C) at a concentration of 0.05%, the chondroitin sulfate being a substrate for the action of the MPDE. The agarose matrix is prepared by mixing powdered agarose with sterile double distilled water, as known per se;

a 40 mg% solution of toluidine blue in a mixture of acetone and double distilled water in a ratio of 5:1 (4 ml of toluidine solution are supplied for each micro-tissue-culture dish);

a 5% acetic acid solution in double distilled water in an amount sufficient to rinse unbound toluidine blue dye off each dish; and

a chart of color patterns.

Example 2

An assay kit is provided as in Example 1, further comprising a set of dishes with different chondroitin sulfate concentrations in the substrate, e.g. 0.01%, 0.05% and 0.1%.

The object of the different concentrations of the chondroitin sulfate is to enable a better determination of the severity and type of the infection. In addition, reaction in the different concentrations will enable better identification of the type of the disease.

## Example 3

An assay kit is provided as in Example 1 in which the substrate in the micro-tissue culture dishes is hyaluronic-acid instead of chondroitin-sulphate.

## Example 4

An assay kit is provided as in Example 3, further comprising a set of microdishes holding substrates with different concentrations of hyaluronic acid e.g. example, 0.001% to 0.1%.

## Example 5

An assay kit is provided as in Example 1 and in addition to dishes holding choindrotin sulfate also dishes containing hyaluronic acid.

The use of specific substrates can serve as a tool for specific differential diagnosis of microorganisms.

## Example 6

An essay kit is provided as in Example 1 wherein the microtissue culture dishes hold an agar or agarose matrix mixed with one of the MPDE substrates of Examples 1-5 and in addition thereto 3.7 g of Brain-Heart extract boullion serving as the growth medium.

## Example 7

Assay kits are provided as in Example 6 but with the dishes holding a matrix with growth medium according to Example IV A.

## Example 8

An assay kit is provided comprising dishes with matrices according to both of Examples 6 and 7.

## Example 9

An assay kit is provided as in Example 6 with a set of matrices prepared at different pH levels.

## Example 10

Assay kits are provided as in Examples 6-9 wherein the matrices also comprises microorganismal growth regulators.

## Claims

1.  An in vitro method of diagnosing infection in by applying a subject, characterized a sample of body fluid taken from the subject onto a matrix which comprises a substrate degradable by mucopolysaccharide degrading enzyme (MPDE), and, after an incubation period, applying to the matrix a dye capable of binding selectively to said substrate or to a degradation product thereof and removing excess dye from the matrix, whereby a color pattern is obtained on the matrix indicative of MPDE in the tested body fluid.

2.  A method according to Claim 1, characterized in that said matrix comprises a substrate degradable by mucopolysaccharide degrading enzyme (MPDE) and a growth medium for nonviral microorganisms.

3.  A method according to Claim 1 or 2 characterized in that said substrate is a mucopolysaccharide-glucosamin glycan selected from the group consisting of hyaluronic acid, chondroitin, chondroitin sulfate A, chondroitin sulfate B, chondroitin sulfate C, chondroitin 4-sulfate, chondroitin 6-sulfate, heparan sulfate, heparin and keratan sulfate.

4.  A method according to any one of Claims 1 to 3, characterized in that the sample of body fluid is tear fluid, cerebral spinal fluid or blood.

5.  A method according to any one of the preceding claims characterized in that a selective growth inhibitor specific for microorganisms is incorporated in the matrix.

6.  A method according to any one of the preceding claims characterized in that the pH of the matrix is adjusted to 4.0 to 8.5.

7.  A method according to any one of the preceding Claims, characterized in that said sample of body fluid which is withdrawn from the subject is absorbed onto an absorbent carrier, preferably of absorbent paper, which carrier is placed onto said matrix.

8.  A method according to Claim 7, wherein said absorbent carrier with the absorbed body fluid is incubated on said matrix for 3 to 24 hours at 37°C and is removed at the end of incubation period prior to the application of said dye.

9. A matrix for use in the method according to any one of Claims 1 to 8, characterized in that it comprises a substrate degradable by MPDE.

10. A matrix according to Claim 9, characterized in that it also comprises a growth medium for non-viral microorganisms.

11. An assay kit for the performance of the method according to any one Claims 1 to 6, comprising at least one capillary tube, at least one piece of an absorbent carrier, characterized in that it comprises at least one dish with a matrix comprising a substrate for MPDE, and a dye capable of binding selectively either to said substrate or to a degradation product thereof.

12. A kit according to Claim 11, characterized in that it comprises a plurality of dishes holding matrices with different substrates for MPDE selected from the group consisting of hyaluronic acid, chondroitin, chondroitin sulfate A, chondroitin sulfate B, chondroitin sulfate C, chondroitin 4-sulfate, chondroitin 6-sulfate, heparan sulfate, heparin and keratan sulfate.

13. A kit according to Claim 11 or 12, characterized in that at least one of said matrices also comprises a growth medium for non-viral microorganisms.

14. A kit according to any one of Claims 11 to 13, characterized in that it comprises at least one dish with a matrix comprising a selective microorganism growth inhibitor.

**Patentansprüche**

1. In vitro-Verfahren zum Diagnostizieren einer Infektion bei einem Patienten, gekennzeichnet durch Aufbringen einer Probe von Körperflüssigkeit, die von dem Patienten entnommen worden ist, auf eine Matrix, die ein Substrat umfaßt, das durch Mucopolysaccharid-abbauendes Enzym (MPDE) abgebaut werden kann, und nach einer Inkubationszeit Aufbringen eines Farbstoffs auf die Matrix, der imstande ist, selektiv an das Substrat oder ein Abbauprodukt davon gebunden zu werden, und Entfernen des überschüssigen Farbstoffs von der Matrix, wodurch ein Farbmuster auf der Matrix erhalten wird, das ein Zeichen ist für das Vorhandensein oder Nichtvorhandensein von MPDE in der untersuchten. Körperflüssigkeit.

2. Verfahren nach Anspruch 1, dadurch gekennzeichnet, daß die Matrix ein Substrat umfaßt, das durch Mucopolysaccharid-abbauendes Enzym (MPDE) abbaubar ist, und ein Wachstumsmedium für nicht-virale Mikroorganismen.

3. Verfahren nach Anspruch 1 oder 2, dadurch gekennzeichnet, daß das Substrat ein Mucopolysaccharid-glucosamin-glycan ist, ausgewählt aus der Gruppe bestehend aus Hyaluronsäure, Chondroitin, Chondroitinsulfat A, Chondroitinsulfat B, Chondroitinsulfat C, Chondroitin-4-sulfat, Chondroitin-6-sulfat, Heparan-sulfat, Heparin und Keratan-sulfat.

4. Verfahren nach einem der Ansprüche 1 bis 3, dadurch gekennzeichnet, daß die Probe von Körperflüssigkeit Tränenflüssigkeit, Cerebral-Spinal-Flüssigkeit oder Blut ist.

5. Verfahren nach einem der vorangehenden Ansprüche, dadurch gekennzeichnet, daß ein selektiver Wachstumsinhibitor, der für die Mikroorganismen spezifisch ist, in die Matrix eingebaut wird.

6. Verfahren nach einem der vorangehenden Ansprüche, dadurch gekennzeichnet, daß der pH-Wert der Matrix auf 4,0 bis 8,5 eingestellt wird.

7. Verfahren nach einem der vorangehenden Ansprüche, dadurch gekennzeichnet, daß die Probe der Körperflüssigkeit, die von dem Patienten entnommen worden ist, auf einem absorbierenden Träger, vorzugsweise absorbierendem Papier, absorbiert wird, wobei der Träger sich auf der Matrix befindet.

8. Verfahren nach Anspruch 7, wobei der absorbierende Träger mit der absorbierten Körperflüssigkeit auf der Matrix 3 bis 24 h bei 37°C inkubiert und am Ende der Inkubationszeit vor dem Aufbringen des Farbstoffs entfernt wird.

9. Matrix zur Verwendung bei dem Verfahren nach einem der Ansprüche 1 bis 8, dadurch gekennzeichnet, daß sie ein durch MPDE abbaubares Substrat umfaßt.

10. Matrix nach Anspruch 9, dadurch gekennzeichnet, daß sie zusätzlich ein Wachstumsmedium für nicht-virale Mikroorganismen umfaßt.

11. Analyseneinheit zur Durchführung des Verfahrens nach einem der Ansprüche 1 bis 6, umfassend mindestens ein Kapillarröhrchen, mindestens ein Stück eines absorbierenden Trägers, dadurch gekennzeichnet, daß sie mindestens eine Schale mit einer Matrix, umfassend ein Substrat für MPDE, und einen Farbstoff, der in der Lage ist, selektiv entweder an das Substrat oder an ein Abbauprodukt davon gebunden zu werden, umfaßt.

12. Analyseneinheit nach Anspruch 11, dadurch gekennzeichnet, daß sie eine Vielzahl von Schalen umfaßt, die Matrices mit unterschiedlichen Substraten für MPDE, ausgewählt aus der Gruppe bestehend aus Hyaluronsäure, Chondroitin, Chondroitinsulfat A, Chondroitinsulfat B, Chondroitinsulfat C, Chondroitin-4-sulfat, Chondroitin-6-sulfat, Heparan-sulfat, Heparin und Keratan-sulfat, enthalten.

13. Analyseneinheit nach Anspruch 11 oder 12, dadurch gekennzeichnet, daß zumindest eine der Matrices zusätzlich ein Wachstumsmedium für nicht-virale Mikroorganismen umfaßt.

14. Analyseneinheit nach einem der Ansprüche 11 bis 13, dadurch gekennzeichnet, daß sie mindestens eine Schale mit einer Matrix, umfassend einen selektiven Mikroorganismen-Wachstumsinhibitor, umfaßt.

## Revendications

1. Procédé in vitro pour diagnostiquer une infection chez un sujet, caractérisé en ce que l'on applique un échantillon de liquide organique prélevé chez le sujet sur une matrice qui comprend un substrat décomposable par une enzyme décomposant les mucopolysaccharides (MPDE) et en ce que, après une période d'incubation, on applique sur la matrice un colorant capable de se lier sélectivement audit substrat ou à un de ses produits de décomposition, et on élimine l'excès de colorant de la matrice, ce qui permet d'obtenir une image colorée sur la matrice, constituant un indicateur de la MPDE dans le liquide organique soumis au dosage.

2. Procédé selon la revendication 1, caractérisé en ce que ladite matrice comprend un substrat décomposable par une enzyme décomposant les mucopolysaccharides (MPDE) et un milieu de culture pour microorganismes non viraux.

3. Procédé selon la revendication 1 ou 2, caractérisé en ce que ledit substrat est un mucopolysaccharide glucosamino-glycane choisi dans le groupe constitué par l'acide hyaluronique, la chondroïtine, la chondroïtine sulfate A, la chondroïtine sulfate B, la chondroïtine sulfate C, la chondroïtine 4-sulfate, la chondroïtine 6-sulfate, l'héparane sulfate, l'héparine et la kératane sulfate.

4. Procédé selon l'une quelconque des revendications 1 à 3, caractérisé en ce que l'échantillon de liquide organique est du liquide lacrymal, du liquide céphalo-rachidien ou du sang.

5. Procédé selon l'une quelconque des revendications précédentes, caractérisé en ce que l'on incorpore dans la matrice un inhibiteur de croissance sélectif, spécifique des microorganismes.

6. Procédé selon l'une quelconque des revendications précédentes, caractérisé en ce que l'on ajuste le pH de la matrice à une valeur de 4,0 à 8,5.

7. Procédé selon l'une quelconque des revendications précédentes, caractérisé en ce que ledit échantillon de liquide organique qui est prélevé chez le sujet est absorbé sur un support absorbant, de préférence de papier absorbant, ce support absorbant étant placé sur ladite matrice.

8. Procédé selon la revendication 7, dans lequel ledit support absorbant portant le liquide organique absorbé est incubé sur ladite matrice pendant 3 à 24 heures à 37°C et est retiré à l'issue de la période d'incubation avant l'application dudit colorant.

9. Matrice à utiliser dans le procédé selon l'une quelconque des revendications 1 à 8, caractérisée en ce qu'elle comprend un substrat décomposable par une MPDE.

10. Matrice selon la revendication 9, caractérisée en ce qu'elle comprend aussi un milieu de culture pour microorganismes non viraux.

11. Trousse de dosage pour réaliser le procédé selon l'une quelconque des revendications 1 à 6, comprenant au moins un tube capillaire, au moins un morceau d'un support absorbant, caractérisée en ce qu'elle comprend au moins une capsule contenant une matrice comprenant un substrat pour la MPDE et un colorant capable de se lier sélectivement ou bien audit substrat, ou bien à un de ses produits de décomposition.

12. Trousse selon la revendication 11, caractérisée en ce qu'elle comprend une pluralité de capsules contenant des matrices avec différents substrats pour la MPDE, choisis dans le groupe constitué par l'acide hyaluronique, la chondroïtine, la chondroïtine sulfate A, la chondroïtine sulfate B, la chondroïtine sulfate C, la chondroïtine 4-sulfate, la chondroïtine 6-sulfate, l'héparane sulfate, l'héparine et la kératane sulfate.

13. Trousse selon la revendication 11 ou 12, caractérisée en ce que l'une au moins desdites matrices comprend aussi un milieu de culture pour microorganismes non viraux.

14. Trousse selon l'une quelconque des revendications 11 à 13, caractérisée en ce qu'elle comprend au moins une capsule contenant une matrice comprenant un inhibiteur sélectif de croissance des microorganismes.

Fig. 1

Fig. 2

Fig. 3

Fig. 4

Fig. 5

Fig. 6

Fig. 7

71          72          73          74

Fig. 8

81,          82,          83

Fig. 9

Fig. 10

Fig. 11

Fig. 12

Fig. 13

Fig. 14

| MICROCOCCUS GRAM+ | STAPH ALBUS | STAPH AUREUS | STREPTOCOCCUS VIRIDANS |

141          142          143          144

Fig. 15

20

Fig. 16

Fig. 17

Fig. 18